# EUROPÄISCHE PATENTSCHRIFT

(11) **EP 1 618 919 B1**
(45) Veröffentlichungstag und Bekanntmachung des Hinweises auf die Patenterteilung: **04.07.2012**
(21) Anmeldenummer: 05105559.8
(22) Anmeldetag: 22.06.2005
(51) Int. Cl.: A61N 1/05

(54) **Fixierung für implantierbare Elektroden und Katheter**
Fixation means for implantable electrodes and catheters
Fixation pour électrodes et cathéters implantables

(30) Priorität: 20.07.2004 DE 102004035903; 21.07.2004 DE 102004035987
(43) Veröffentlichungstag der Anmeldung: 25.01.2006
(73) Patentinhaber: Biotronik CRM Patent AG, 6340 Baar/Zug (CH)
(72) Erfinder: Flach, Erhard, 12305 Berlin (DE); Geistert, Wolfgang, 79618 Rheinfelden (DE); Kolberg, Gernot, 12043 Berlin (DE); Kuttler, Dr. Marc, 12205 Berlin (DE)
(74) Vertreter: Lindner-Vogt, Karin L.

(56) Entgegenhaltungen:
- EP-A1- 0 085 967
- EP-A1- 0 337 035
- EP-A2- 0 966 979
- WO-A1-01/08745
- US-A- 5 931 864

## Beschreibung

Die Erfindung betrifft eine Fixierung für implantierbare Elektroden und Katheter.

Aktive elektronische Implantate für die Funktionelle Elektrostimulation (FES) müssen in der Regel am Ort des Einsatzes verankert werden, damit ihre für Therapie oder Diagnose gewünschte Position im Gewebe über die Zeit beibehalten wird. Besonders wichtig ist dies für implantierbare Elektroden oder für über einen längeren Zeitraum aufgelegte Katheter, die in einer bewegten Organmatrix, z. B. im Herzen, angeordnet sind. Für die Fixierung wurde eine Vielzahl unterschiedlichster Lösungen entwickelt. Man unterscheidet Elektroden bzw. Katheter mit passiver Fixierung (z.B. widerhakenähnliche Ankersysteme aus dem Isolationsmaterial der Elektrode / des Katheters) von solchen mit aktiver Fixierung (z.B. Schraubsystem am Elektrodenkopf). Erstere eignen sich insbesondere für die Verankerung in der stark gegliederten Oberfläche der rechten Herzkammer (Trabekelstruktur), letztere insbesondere für die Verankerung im glatteren rechten Herzvorhof. Alle bekannten Fixierungen werden aus Werkstoffen geformt, die möglichst biokompatibel und bioresistent (nicht in vivo abbaubar) sind.

Zum bestimmungsgemäßen Gebrauch werden die Elektroden / die Katheter zunächst am gewünschten Ort im Körper des Patienten positioniert, mit geeigneten Mitteln verankert und verbleiben dann je nach therapeutischer oder diagnostischer Fragestellung für einen bestimmten Zeitraum in der vorgegebenen Position. Nach Abschluss der Therapie / Diagnose oder aus anderen Gründen (z.B. Batteriewechsel) muss die Elektrode / der Katheter wieder entfernt werden. Dementsprechend sind an die Fixierung über die Zeit unterschiedlichste Anforderungen zu stellen.

Während der Positionierung der Elektrode / des Katheters ist eine Fixierung, die sperrig ist oder schon große Haltekräfte entwickelt, unerwünscht, da dies die Positionierung als auch eine Repositionierung (zur Korrektur einer fehlerhaften Positionierung) behindert und zu Gewebsverletzungen führen kann. Das Problem stellt sich besonders bei passiven Fixierungen.

Weiterhin verändert sich mit zunehmender Verweildauer der implantierbaren Elektrode / des Katheters im Körper durch allmähliches Einwachsen die Haltekraft der Fixierung. So kann die Haltekraft kurz nach der Positionierung noch relativ gering sein, so dass die Gefahr einer unerwünschten Repositionierung gegeben ist. Dagegen lassen sich die Elektroden / die Katheter nach längerer Verweildauer im Körper nicht mehr ohne größere Eingriffe entfernen, denn mit zunehmendem Einwachsen steigen auch die Haltekräfte zwischen Fixierung und Gewebsumgebung.

Dokument US-A-5 931 864 offenbart den Oberbegriff des Anspruchs 1.

Aufgabe der vorliegenden Erfindung ist es, eine Fixierung bereitzustellen, die den unterschiedlichen Anforderungen im zeitlichen Verlauf des bestimmungsgemäßen Gebrauchs der implantierbaren Elektroden bzw. der Katheter besser Rechnung trägt.

Diese Aufgabe wird durch die erfindungsgemäße Fixierung für implantierbare Elektroden und Katheter nach Anspruch 1 gelöst. Die Fixierung zeichnet sich dadurch aus, dass sie wenigstens eine erstes Strukturelement umfasst, das aus einem biodegradierbaren Werkstoff besteht. Durch den allmählichen Abbau des ersten Strukturelementes kann ein über die Zeit flexibleres Verhalten der Fixierung erreicht werden, denn die durch den Abbau bedingten Veränderungen beeinflussen auch die Haltekraft der Fixierung. Somit steht dem mit der Ausgestaltung von Fixierungen befassten Fachmann - einem Ingenieur mit vertieften Kenntnissen auf dem Gebiet implantierbarer medizinischer Systeme - ein weiterer Parameter zur Optimierung der Fixierungseigenschaften zur Verfügung. Je nach Elektroden- oder Kathetertyp und geplanter Anwendung können die Fixierungseigenschaften angepasst werden.

Unter "Strukturelement" im erfindungsgemäßen Sinne wird ein dreidimensionales Gebilde verstanden, das zumindest einen Teil der Fixierung bildet, der zumindest temporär zur Höhe einer Haltekraft, die zwischen der Fixierung und dem umgebenden Gewebe besteht, beiträgt. Der Abbau des ersten Strukturelementes beeinflusst die Haltekraft der Fixierung, wobei die "Haltekraft" die Kraft ist, die notwendig ist, um die Elektrode bzw. den Katheter in proximaler Richtung der Elektrodenleitung bzw. elektrischen Zuleitung wieder aus dem Gewebe zu entfernen, in dem es bei bestimmungsgemäßen Gebrauch angeordnet ist. Die Höhe der Haltekraft ist abhängig von den mechanischen Eigenschaften der Fixierung und des umgebenden Gewebes. Somit kann durch den Abbau des ersten Strukturelementes die Haltekraft erhöht oder erniedrigt werden, aber auch eine Änderung der Haltekraft über die Zeit durchs Gewebsveränderungen kompensiert werden.

Ein Strukturelement kann aktiv oder passiv ausbildet sein. "Aktiv" bedeutet, dass die zur Fixierung notwendige Lage und/oder Raumform des Strukturelementes erst durch Einwirken äußerer Kräfte eingenommen wird. So kann z. B. ein schraubenförmiges Strukturelement während der Positionierung mittels eines Schraubenmandrins vom Chirurgen aus dem Elektrodenkopf herausgedreht und im Gewebe verankert werden. Unter "passiven Strukturelementen" werden dreidimensionale Gebilde verstanden, die aufgrund ihrer Lage und/oder Raumform zumindest temporär einen Halt im Gewebe gewähren. Passive Strukturelemente umfassen z. B. nadel-, haken- oder ankerförmige Elemente, Vorsprünge (Hinterschneidungen) auf der Elektrodenleitung oder dem Katheterkörper sowie zick-zack- oder wendelförmige Herzdrähte. Aktive und passive Strukturelemente können miteinander kombiniert werden.

Das erste Strukturelement ist aus einem biodegradierbaren Wirkstoff geformt. Unter "biodegradierbar" im Sinne der Erfindung wird ein Werkstoff verstanden, der in physiologischer Umgebung durch korrosive Prozesse ganz oder zumindest weitestgehend abgebaut wird. Der Abbau kann dabei unter anderem durch Wahl des Werkstoffs, Aufbringen von Beschichtungen, Geometrie der Fixierung, Strömungsverhältnisse im Gewebe und Morphologie des Werkstoffs sowohl im Zeitpunkt des Einsetzens oder vollständigen Abschlusses, als auch im seinem zeitlichen Verlauf beeinflusst werden. Der Abbau kann sehr rasch erfolgen (z.B. innerhalb weniger Minuten) oder, wenn gewünscht, auch über mehrere Jahre (z.B. bis zu zehn Jahre) hinaus gezögert werden. Die Werkstoffe als auch die Abbauprodukte sollten möglichst biokompatibel sein.

Aufgrund der mechanischen Eigenschaften und der hohen Biokompatibilität ist erfindungsgemäß der Einsatz einer biodegradierbaren Legierung auf Basis von Magnesium vorgesehen. Der Anteil der Hauptkomponente Magnesium an den Legierungen beträgt mindestens 50 Gew.%, bevorzugt mindestens 70 Gew.%, besonders bevorzugt mindestens 90 Gew.%. Hierdurch ist ein weitestgehender Abbau des ersten Strukturelementes und eine weitestgehende Resorption der Abbauprodukte im Körper sichergestellt.

Besonders bevorzugt sind biodegradierbare Magnesiumlegierungen, die Seltenerdmetalle und Yttrium enthalten, wobei die Sammelbezeichnung 'Seltenerdmetall' für die Elemente Scandium (Ordnungszahl 21), Lanthan (57) sowie die 14 auf das Lanthan folgenden Elemente Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), die als Lanthanoide bezeichnet werden, steht. Besonders bevorzugt weisen die Magnesiumlegierungen folgende Gewichtsanteile der Legierungskomponenten auf:
- Seltenerdmetalle 2.0 bis 5.0 Gew.% und/oder
- Yttrium 3.5 bis 4.5 Gew.% und/oder
- Neodym 1.5 bis 3.0 Gew.% und/oder
- Zirkonium 0.3 bis 1.0 Gew.% und/oder
- Aluminium < 0.5 Gew.%, insbesondere < 0.01 Gew.%, und/oder
- Rest < 0.5 Gew.%, insbesondere < 0.3 Gew.%,
wobei Magnesium den auf 100 Gew.% verbleibenden Gewichtsanteil an der Legierung einnimmt. Die genannten Magnesiumlegierungen zeigen ein günstiges Abbauverhalten, hohe Biokompatibilität der Legierung als auch der Abbauprodukte und besitzen für das Einsatzgebiet ausreichende mechanische Eigenschaften.

Nach einer Variante umfasst die Fixierung ein zweites Strukturelement aus einem bioresistenten Werkstoff, das zumindest einen Teil der Fixierung bildet, der zumindest temporär zur Höhe einer Haltekraft, die zwischen der Fixierung und dem umgebenden Gewebe besteht, beiträgt. Das zweite Strukturelement trägt demnach zur Haltekraft der Fixierung bei - sei es vor, während oder erst nach dem Abbau des ersten Strukturelementes. Hierdurch können die Fixierungseigenschaften, insbesondere ein zeitlicher Verlauf der Haltekraft, vom zuständigen Fachmann in einer an die jeweiligen Erfordernisse möglichst optimierten Art und Weise vorgegeben werden. So kann beispielsweise vorgesehen sein, dass passive Strukturelemente auch nach dem vollständigen Abbau des ersten Strukturelementes vorhanden sind und einen Betrag zur Haltekraft leisten.

In einer Variante ist vorgesehen, dass das zweite Strukturelement zumindest einen ersten und zweiten Zustand einnehmen kann, wobei der erste Zustand eine für die Positionierung der Elektrode bzw. des Katheters günstige Raumform und/oder Lage des zweiten Strukturelementes und der zweite Zustand eine für die Fixierung der Elektrode bzw. des Katheters günstige Raumform und/oder Lage des zweiten Strukturelementes einnimmt. Weiterhin ist das zweite Strukturelement so ausgebildet, dass der erste Zustand ohne äußeren Zwang in den zweiten Zustand übergeht. Das erste Strukturelement ist zudem so ausgebildet, dass es vor dem Einsetzen der Abbauprozesse das zweite Strukturelement im ersten Zustand fixiert. Das erste Strukturelement umgreift bzw. umschließt somit das zweite Strukturelement derart, dass erst nach Abbau oder weitestgehendem Abbau der für die Fixierung gewünschte zweite Zustand eingenommen werden kann. Der Übergang vom ersten in den zweiten Zustand kann durch mechanisches Vorspannen, aber auch mit Hilfe von Gedächnislegierungen erreicht werden. Hierbei kann insbesondere zur konkreten Umsetzung des zweiten Strukturelementes Rückgriff auf die Formvielfalt und Materialwahl bekannter selbstexpandierender Stents erfolgen.

Nach einer zweiten Variante einer Fixierung, die ein bioresistentes zweites Strukturelemente beinhaltet, ist umfasst das zweite Strukturelement ein netzförmiges, gitterartiges oder schwammartiges (poröses) Grundgerüst. Das erste Strukturelement kann nun dieses Grundgerüst bedecken oder die Freiräume im Grundgerüst füllen. Mit dem allmählichen Abbau des ersten Strukturelementes wird dieses Grundgerüst von außen zugänglich, mit der Folge, dass das umliegende Gewebe Einwachsen kann. Dementsprechend sind die Strukturen des Grundgerüsts, d.h. Öffnungen, Poren, Aussparungen oder dergleichen, in ihren Abmessungen so auszulegen, dass das Gewebe einwachsen kann. Diese zweite Variante kann beispielsweise derart umgesetzt werden, dass ein poröses Grundgerüst als zweites Strukturelement vorgegeben wird, dessen Poren mit einer biodegradierbaren Magnesiumlegierung verschlossen sind (das Füllmaterial entspricht damit dem ersten Strukturelement). Mit Einsetzen und nach Abschluss der Abbauprozesse werden die Poren wieder frei gegeben und das umliegende Gewebe kann in die entstehenden Freiräume einwachsen. Denkbar ist aber auch, dass das biodegradierbare erste Strukturelement nicht einen Zugang des Gewebes zu den Freiräumen des Grundgerüsts des zweiten Strukturelementes verwehrt, sondern an anderer Stelle der Elektrode angeordnet ist. Der Vorgang des Einwachsens des umliegenden Gewebes in das zweite Strukturelement kann demnach schon unmittelbar nach der Implantation einsetzten, es dauert aber bekanntermaßen einige Tage bis dieser Vorgang abgeschlossen ist. In dieser Zeit kann der Beitrag des biodegradierbaren ersten Strukturelements zur Haltekraft eine unerwünschte Repositionierung verhindern.

Nach einer dritten Variante der Fixierung, die ein bioresistentes zweites Strukturelemente beinhaltet, ist selbiges nicht mehr unmittelbar an die Elektrode bzw. den Katheter gebunden. Vielmehr bildet das erste Strukturelement eine Verbindung zwischen der Elektrode oder dem Katheter und dem zweiten Strukturelement. Durch allmählichen Abbau des ersten Strukturelementes wird die Verbindung zum zweiten Strukturelement geschwächt bzw. letztendlich gänzlich aufgehoben. Dementsprechend verringert sich ein Anteil des zweiten Strukturelementes an der Haltekraft im Laufe der Zeit. Nach der Trennung oder weitestgehenden Aufhebung der Verbindung zwischen dem ersten und zweiten Strukturelement kann die Elektrode sehr viel einfacher und in der Regel ohne Komplikationen entfernt werden, wobei das zweite Strukturelement im Körper verbleibt.

Nach der erfindungsgemäßen Fixierung ist vorgesehen, dass die Fixierung eine elektrische Zuleitung umfasst, die mit dem ersten Strukturelement elektrisch leitend verbunden ist und über die an das erste Strukturelement eine elektrische Spannung angelegt werden kann. Durch Anlegen einer elektrischen Spannung zwischen der Zuleitung zur Fixierung und einer Gegenelektrode, z. B. dem Implantatsgehäuse oder einem elektrischen Pol der Elektrode, kann der Abbauprozess bei Bedarf beschleunigt werden.

Bekannt sind eine Vielzahl verschiedener Fixierungseinrichtungen mit Fixierungselementen, wie Schrauben, Nadeln, Haken, Anker (so genannte Tines) sowie viele Arten von Vorsprüngen, welche eine Hinterschneidung bilden, in die Gewebe einwachsen kann, wodurch eine Fixierung erreicht wird.
Diese Fixierungselemente haben, um ihre Funktion erfüllen zu können, oft scharfe Spitzen oder Kanten. Diese scharfen Spitzen oder Kanten können ungewollte Verletzungen hervorrufen, wenn eine solche Elektrode oder ein solcher Katheter durch eine kleine Öffnung oder durch ein Blutgefäß in den Körper eingeführt und zum Bestimmungsort geführt wird.

Es ist deshalb zweckmäßig, diese Fixierungselemente für den Einführvorgang mittels eines temporären Schutzelementes, z.B. einer Kapsel, zu schützen. Dieses Schutzelement muss sich im Körper auflösen, wodurch die Fixierungselemente freigelegt werden.

Lösliche Kapseln sind z.B. aus US 4,827,940 und aus EP 0337035 bekannt. In diesen Schriften wird vorgeschlagen, die Kapseln aus zuckerverwandten Materialien, wie Mannitol, Dextrose, Sorbose, Sucrose, oder aus Salzen, wie Natriumchlorid, Kaliumchlorid oder Natriumkarbonat, oder aus einem gelbildenden Material, wie Gelantine, hydrophilen Polymeren, vernetztem Polyethylen-Glykol (PEG), Zellulose, Dextran usw. herzustellen.

All diesen Materialien ist gemeinsam, dass sie entweder relativ spröde oder sehr weich sind, so dass sie die Fixierungselemente nur sehr begrenzt schützen, da sie sich bereits beim Einführprozess lösen könnten. Außerdem ist es mit diesen Materialien nicht möglich, ein Schutzelement herzustellen, das ein Fixierungselement, welches federnde Bestandteile enthält, in einer anderen, für die Einführung in den Körper besser geeigneten Form hält.

Von Vorteil wären deshalb Schutzelemente, die - bevor sie sich auflösen - die Fixierungselemente wirksamer schützen und/oder in einer für den Einführvorgang günstigeren Form halten können.

Dies wird dadurch erreicht, dass das erste Strukturelement ein Schutzelement ist, das im nicht implantierten Zustand ein zur Fixierung dienendes zweites Strukturelement ganz oder in Teilen umgreift und das ganz oder zu mindestens 70 Gew.% aus der biodegradierbaren Magnesiumlegierung besteht.

Die mechanischen Eigenschaften des Werkstoffs, wie Bruchfestigkeit, Sprödigkeit und E-Modul sowie seine leichte Verarbeitbarkeit zeichnen sich für den angestrebten Anwendungszweck besonders aus. Zudem zeigt der Werkstoff als seine Abbauprodukte eine hohe Biokompatibilität, teils einhergehend mit positiven physiologischen Effekten auf das umgebende Gewebe. Der Abbau des Werkstoffs erfolgt rasch innerhalb weniger Stunden bis Tage.

Vorzugsweise besteht das Schutzelement zu mindestens 80 Gew.%, insbesondere 90 Gew.%, aus dem biodegradierbaren Werkstoff. Die nach dem Abbau verbleibenden Restbestandteile vermögen nicht mehr die mechanischen Eigenschaften des Schutzelementes aufrecht zu erhalten und verbleiben zumeist als feines Pulver im Körper, ohne dass es zu wesentlichen Gewebsirritationen kommt. Dementsprechend sind diese nicht degradierbaren Bestandteile biokompatibel auszulegen.

Ein Umgreifen der Fixierelemente (des zweiten Strukturelementes) durch das Schutzelement, sei es ganz oder in Teilen, ist so zu verstehen, dass die Teilstrukturen der Fixierung, die bei der Positionierung Gewebsverletzungen beifügen könnten, vom Schutzelement abgedeckt werden und nicht mehr mit den umgebenden Gewebe in unerwünschter Weise Wechselwirken können. Dieses schützende Umgreifen schließt den Gedanken ein, die Fixierung auch in einer für die Positionierung günstigeren Form durch das Schutzelement zu halten.

In einer ersten Variante ist ein solches Schutzelement so gestaltet, dass es die zur Fixierung notwendigen zweiten Strukturelemente, insbesondere Spitzen, Kanten, Vorsprünge, der Fixierung ganz oder teilweise so umschließt, so dass selbige nicht mehr frei liegen und das Gewebe bei der Positionierung unbeabsichtigt verletzen könnten.

In einer zweiten Variante hält das Schutzelement die zur Fixierung notwendigen zweiten Strukturelemente der Fixierung in einer für den Einführvorgang günstigeren Form. Sobald das Schutzelement abgebaut ist geht das zweite Strukturelement in eine zweite Form über, die zur Fixierung im umliegenden Gewebe günstiger ist.

Ein solches Schutzelement kann aus einer relativ dünnen Wandung bestehen, die einen Hohlraum umschließt. Ein solches Schutzelement kann aber auch aus massivem Material oder aus aufgeschäumten Material bestehen, in welches das zweite Strukturelement eingebettet ist. Schließlich kann ein solches Schutzelement auch aus einer relativ offen gestalteten Draht- oder Bandkonstruktion bestehen. Das Schutzelement kann die Form einer Kapsel, einer Klammer oder eines Rings annehmen, um das zweite Strukturelement zu schützen.

Die Erfindung wird nachfolgend anhand von Ausführungsbeispielen und den dazugehörigen Zeichnungen näher erläutert. Es zeigen:
Fig. 1 eine Draufsicht einer Elektrode im Bereich einer Fixierung, die ein expandierbares Strukturelement umfasst;
Fig. 2a und 2b eine Draufsicht und einen Querschnitt durch eine Elektrode im Bereich einer Fixierung, bei der die Elektrode am Außenumfang eines expandierbaren Strukturelementes befestigt ist;
Fig. 3 eine Draufsicht auf eine Elektrode im Bereich einer Fixierung, die ein Strukturelement in Form eines Fixierungsankers umfasst;
Fig. 4 eine Draufsicht auf einen Katheter im Bereich einer Fixierung, die ein zylindrisch-sägezahnförmiges Strukturelement umfasst;
Fig. 5 eine Draufsicht auf eine Elektrode im Bereich einer Fixierung, die ein Strukturelement in Form eines Schraubwendel umfasst;
Fig. 6 eine Draufsicht auf eine Elektrode im Bereich einer Fixierung, die ein Strukturelement in Form eines zick-zack-förmigen Herzdrahts umfasst;
Fig. 7 eine Draufsicht auf eine Elektrode im Bereich einer Fixierung, die ein Strukturelement in Form eines wendelförmigen Herzdrahts umfasst;
Fig. 8 eine rückseitige Draufsicht auf einen Querschnitt durch eine Elektrode unterhalb der Fixierung, die ein Strukturelement in Form eines Fixierungsankers umfasst;
Fig. 9 einen Längsschnitt durch eine Elektrode im Bereich einer Fixierung, die ein Strukturelement in Form eines Fixierungsankers umfasst;
Fig. 10 ein Längsschnitt durch eine Elektrode im Bereich einer Fixierung, die ein zylindrisch-sägezahnförmiges Strukturelement umfasst;
Fig. 11 a und 11 b eine Draufsicht und ein Längsschnitt durch eine Elektrode im Bereich einer Fixierung, die einen porösen Grundkörper als Strukturelement umfasst;
Fig. 12 eine Draufsicht auf eine Elektrode im Bereich einer Fixierung, die ein Strukturelement in Form eines Fixierungsanker und eines porösen Grundkörpers umfasst;
Fig. 13 einen Längsschnitt durch einen Katheter im Bereich einer Fixierung, die ein zylindrisch-sägezahnförmiges Strukturelement umfasst;
Fig. 14 ein Längsschnitt durch eine Elektrode im Bereich einer Fixierung, die ein Strukturelement in Form eines Fixierungsankers umfasst, der über eine Verbindung an dem Elektrodenkörper haftet;
Fig. 15a und 15b eine Draufsicht und einen Querschnitt durch eine Elektrode im Bereich einer Fixierung, die ein expandierbares Strukturelement umfasst;
Fig. 16 zeigt die Spitze einer Elektrode mit einer ein Schutzelement enthaltenden Fixierung;
Fig. 17 zeigt die Spitze einer Elektrode mit einer ein Schutzelement enthaltenden Fixierung nach einer zweiten Alternative;
Fig. 18a und 18b zeigen die Spitze einer Elektrode mit einer ein Schutzelement enthaltenden Fixierung in einer dritten Alternative und
Fig. 19a und 19b zeigen die Spitze einer Elektrode mit einer ein Schutzelement enthaltenden Fixierung in einer vierten Alternative.

Nachfolgend werden in mehreren Ausführungsbeispielen erfindungsgemäße Fixierungen in unterschiedlichster Ausgestaltung beschrieben. Ein Schutzumfang der Ansprüche soll jedoch nicht auf die konkreten Ausführungsbeispiele beschränkt sein, sondern diese dienen lediglich der Illustration des Erfindungsgedankens. Gemeinsam ist den erfindungsgemäßen Fixierungen, dass sie wenigstens ein erstes Strukturelement umfassen, das aus einem biodegradierbaren Werkstoff besteht, nämlich einer biodegradierbaren Magnesiumlegierung mit folgenden Gewichtsanteilen:
- Seltenerdmetalle 2.0 - 5.0 Gew. % und
- Yttrium 3.5 - 4.5 Gew. % und
- Neodym 1.5 - 3.0 Gew. % und
- Zirkonium 0.3 - 1.0 Gew. % und
- Aluminium < 0.01 Gew. % und
- Rest < 0.3 Gew. %,
wobei Magnesium den auf 100 Gew. % verbleibenden Anteil an der Legierung bildet. Die genannte Magnesiumlegierung besitzt für die Umsetzung der nachfolgend in den Ausführungsbeispielen beschriebenen Fixierungen besonders geeignete Materialeigenschaften. Weiterhin zeigt die Legierung als auch deren Abbauprodukte eine hohe Biokompatibilität. Zudem scheinen die Abbauprodukte bzw. die Abbauprozesse in vivo einen positiven physiologischen Effekt auf das umgebende Gewebe auszuüben, so dass Abstoßungsreaktionen zumindest gemindert werden. Alle im folgenden beschriebenen Ausführungsbeispiele können auf Basis der vorgenannten Magnesiumlegierung hergestellt werden. Denkbar ist aber auch, die weiter oben beschriebenen alternativen biodegradierbaren Werkstoffe in Kombination oder ergänzend einzusetzen. Gemeinsam ist allen für die Umsetzung des ersten Strukturelementes genutzten Werkstoffen, dass sie in vivo abgebaut werden. Dementsprechend ändert sich über die Zeit ein Beitrag des ersten Strukturelementes zur Haltekraft der Fixierung.

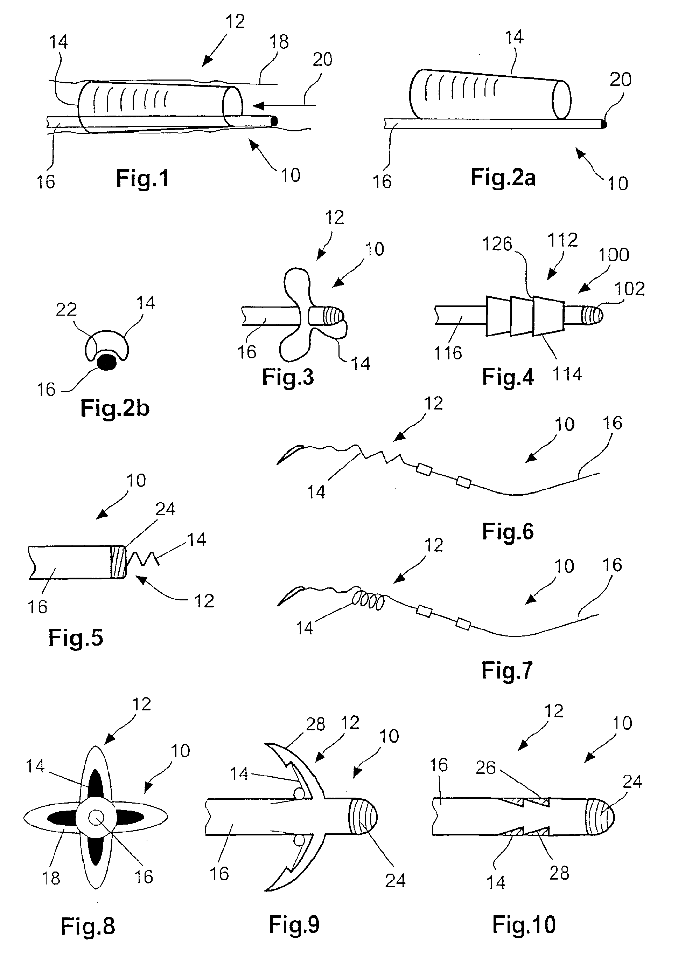

Fig. 1 zeigt in einer schematischen Draufsicht einen Abschnitt aus einer Elektrode 10, und zwar im Bereich des distalen Endes der Elektrodenleitung 16, in dem die Fixierung 12 angeordnet ist. Die Fixierung 12 umfasst ein rohrförmiges, erstes Strukturelement 14, das aus dem biodegradierbaren Werkstoff geformt ist. Das erste Strukturelement 14 kann einen ersten Zustand einnehmen, in dem es einen geringeren und somit für die Positionierung im Körper des Patienten günstigeren Querschnitt aufweist. Am Implantationsort wird das erste Strukturelement 14 durch Expansion in einen zweiten Zustand überführt. Die Fixierung 12 umfasst dazu - hier nicht näher dargestellte - Expansionsmittel, wie z. B. einen an der Elektrodenleitung 16 angeordneten inflatierbaren Ballon. Der konkrete Aufbau des ersten Strukturelementes 14 als auch der Expansionsmittel kann sich an bekannte Lösungen anlehnen, die auf dem Gebiet implantierbarer Gefäßstützen (Stents) entwickelt wurden. Im expandierten zweiten Zustand stützt sich das erste Strukturelement 14 an den hier schematisch angedeuteten Gefäßwänden 18 ab und trägt damit zur Haltekraft der Fixierung 12 bei. Unter Haltekraft wird die Kraft verstanden, die notwendig ist, die Elektrode 10 wieder in proximaler Richtung der Elektrodenleitung 16 (angedeutet durch den Pfeil 20) zu entfernen. Es versteht sich, dass mit zunehmendem Abbau des ersten Strukturelementes 14 dessen Beitrag zur Haltekraft sinkt. Andererseits wächst die Elektrode 10 gleichzeitig in das umgebende Gewebe ein, so dass dessen Beitrag zur Haltekraft steigt.

Bei der in Fig. 1 vorgesehenen Fixierung liegt die Elektrode 10 mit der Elektrodenleitung 16 im Inneren des expandierbaren ersten Strukturelementes 14. Alternativ kann eine Anordnung gemäß den Zeichnungen der Fig. 2a und 2b erfolgen, bei der die Elektrodenleitung 16 am Außenumfang des ersten Strukturelementes 14, insbesondere in einer hierfür vorgesehenen halbrunden, länglichen Vertiefung 22 angeordnet ist. Bei der Expansion des im Aufbau analog zur Fig. 1 ausgeführten, ersten Strukturelementes 14 wird die Elektrodenleitung 16 samt Elektrodenkopf 24 an die Gefäßwand gedrückt.

Fig. 3 zeigt eine Elektrode 10 im Bereich ihrer Fixierung 12. Die Fixierung 12 umfasst ein erstes Strukturelement 14, das die Form eines Fixierungsankers annimmt, der aus einem Draht des biodegradierbaren Werkstoffs hergestellt wird.

In Fig. 4 ist ein endständiger Bereich eines Katheters 100 dargestellt, der einen Elektrodenkopf 102 und eine Fixierung 112 trägt. Die Fixierung 112 umfasst ein erstes Strukturelement 114 aus dem biodegradierbaren Werkstoff, das eine zylindrisch-sägezahnförmige Kontur besitzt, welche in proximaler Richtung einer elektrischen Zuleitung 116 des Katheters 100 Hinterschnitte 126 bildet.

Fig. 5 zeigt ein distales Ende einer Elektrodenleitung 16 einer Elektrode 10, der eine aktive Fixierung 12 umfasst. Aktiv bedeutet, dass die zur Fixierung notwendige Lage des ersten Strukturelementes 14 erst durch Einwirken äußerer Kräfte eingenommen wird. Konkret wird im vorliegenden Fall ein - hier nicht dargestellter - Schraubenmandrin über ein Lumen in der Elektrodenleitung 16 an die Rückseite der Fixierung 12 geführt und greift dort in - ebenfalls nicht dargestellte - komplementäre Strukturen ein. Durch eine Drehung des Schraubenmandrins wird das erste Strukturelement 14 aus dem Elektrodenkopf 24 herausgedreht und verankert sich im anliegenden Gewebe. Das als Schraubwendel ausgeformte erste Strukturelement 14 besteht aus dem biodegradierbaren Werkstoff.

Die Fig. 6 und 7 zeigen schematisch zwei unterschiedliche Varianten zweier Herz-Elektroden 10, jeweils wieder in einem Ausschnitt, der ein distales Ende der Elektrodenleitung 16 mit Fixierung 12 wiedergibt. Wie ersichtlich, umfasst die Fixierung 12 beider Varianten passive, erste Strukturelemente 14 in Form eines sogenannten Herzdrahtes. Bei der Variante gemäß Fig. 6 ist dieser Herzdraht zick-zackförmig und bei der Variante gemäß Fig. 7 wendelförmig ausgeführt. Das erste Strukturelement 14 ist aus dem biodegradierbaren Werkstoff geformt.

Fig. 8 liefert eine rückseitige Ansicht auf das distale Ende einer Elektrode 10 und zwar entlang eines Schnittes durch die Elektrodenleitung 16 knapp unterhalb der Fixierung 12. Selbige umfasst ankerförmige erste und zweite Strukturelemente 14, 28. Das zweite Strukturelement 28 ist aus einem bioresistenten Werkstoff, z. B. dem Isoliermaterial der Elektrodenleitung 16, geformt. In einigen Abschnitten ist das ankerförmige zweite Strukturelement 28 zur Verstärkung mit einem biodegradierbaren, ersten Strukturelement 14 verbunden bzw. beschichtet. Durch den allmählichen Abbau des ersten Strukturelementes 14 nach der Implantation der Elektrode 10 verringert sich ein Beitrag der Strukturelemente 14, 28 zur Haltekraft, den ihr mechanischer Widerstand gegen eine Verschiebung in proximaler Richtung der Elektrodenleitung 16 wird mit zunehmenden Materialabbau sinken.

Fig. 9 stellt einen Längsschnitt durch eine Elektrode 10 mit einer weiteren alternativen Variante seiner Fixierung 12 dar. Die Fixierung 12 setzt sich wiederum aus einem ersten Strukturelement 14 aus dem biodegradierbaren Werkstoff und einem zweiten Strukturelement 28 aus einem bioresistenten Werkstoff zusammen. Das zweite Strukturelement 28 bildet eine ankerförmige Struktur, die auf ihrer zur Elektrodenleitung 16 gewandeten Seite durch das erste Strukturelement 14 verstärkt wird. Das erste Strukturelement 14 ist ein biodegradierbarer Draht, der sich an vorgegebenen Stellen des zweiten Strukturelementes 28 bzw. der Elektrodenleitung abstützt und so zur Haltekraft beiträgt. Das zweite Strukturelement 28 kann beispielsweise aus dem Isolatormaterial der Elektrodenleitung 16, in der Regel einen Silikon, geformt werden. Wenn das erste Strukturelement 14 zumindest weitestgehend abgebaut ist, sinkt ein mechanischer Widerstand der Fixierung 12 gegen eine Verlagerung in proximaler Richtung der Elektrodenleitung 16.

Die Elektrode 10 der Fig. 10 weist eine Fixierung 12 mit zylindrisch-sägezahnförmigen zweiten Strukturelementen 28, die Hinterschnitte 26 in Richtung des proximalen Endes der Elektrode 10 bilden, auf. Die zylindrisch-sägezahnförmigen Vertiefungen des zweiten Strukturelementes 28 sind mit einem eine komplementäre Kontur aufweisenden ersten Strukturelement 14 gefüllt, das aus dem biodegradierbaren Werkstoff besteht. Während und nach Abbau des ersten Strukturelementes 14 wächst das umgebene Gewebe in die sich ergebenden Freiräume ein, so dass sich die Haltekraft in der Regel erhöht.

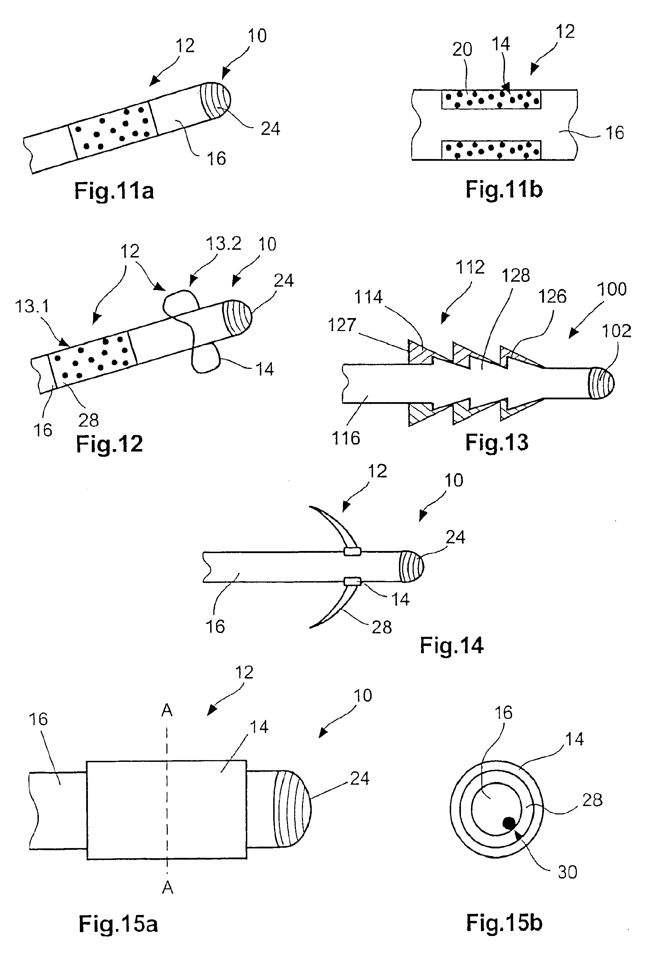

Die Fig. 11a und 11b zeigen in einer Draufsicht bzw. einem Längsschnitt einen distalen Abschnitt einer Elektrode 10 mit einer Fixierung 12. Selbige hat eine hülsenförmige Kontur und liegt in einer dazu komplementären, umlaufenden Aussparung im Isoliermaterial der Elektrodenleitung 16. Die Fixierung 12 setzt sich aus einem ersten und zweiten Strukturelement 14, 28 zusammen. Das zweite Strukturelement 28 beinhaltet ein starres, bioresistentes Grundgerüst, das eine Vielzahl von Poren aufweist. Diese Poren sind vor Implantation mit dem ersten Strukturelement 14 aus einem biodegradierbaren Werkstoff gefüllt. Im Körper wird das erste Strukturelement 14 abgebaut und das umliegende Gewebe kann in die dann freiliegenden Poren einwachsen. Dementsprechend wird eine Dimensionierung der Poren vorgegeben.

Die Fig. 12 gibt eine Elektrode 10 wieder, deren Fixierung 12 eine Kombination der in den Fig. 11 a und 11 b sowie Fig. 3 dargestellten ersten und zweiten Strukturelemente 14, 28 nutzt. Ein proximal gelegener Teil 13.1 der Fixierung 12 umfasst ein zweites Strukturelement 28, das aus einem bioresistenten Werkstoff geformt ist und ein wie in den Fig. 11a und 11b dargestelltes Grundgerüst bildet. Es wird insofern auf die dortigen Ausführungen verwiesen. Weiterhin umfasst die Fixierung 12 einen distalen Teil 13.2 mit einem ankerförmigen ersten Strukturelement 14 aus dem biodegradierbaren Werkstoff. Während des Einwachsens des umliegenden Gewebes in das Grundgerüst des zweiten Strukturelementes 28 trägt das erste Strukturelement 14 wesentlich zur Haltekraft bei. Nach dem Abbau desselben lässt sich die Elektrode 10 wegen der günstigeren isodiametrischen Form des zweiten Strukturelementes 28 in proximaler Richtung ohne große Komplikationen entfernen.

Fig. 13 zeigt einen Längsschnitt durch einen Katheter 100 im Bereich einer Fixierung 112, die ein biodegradierbares erstes Strukturelement 114 und ein bioresistentes zweites Strukturelement 128 beinhaltet. Beide Strukturelemente 114, 128 besitzen eine zylindrisch-sägezahnförmige Kontur, die in proximaler Richtung der elektrischen Zuleitung 116 des Katheters 100 Hinterschnitte 126 bzw. 127 bildet. Die Hinterschnitte 126 des zweiten Strukturelementes 128 sind in ihren Abmessungen kleiner ausgelegt, als die Hinterschnitte 127 des ersten Strukturelementes 114. Somit wird sich der Beitrag der Fixierung 112 zur Haltekraft im Laufe der Zeit verringern.

In der Fig. 14 ist in einem Längsschnitt eine Elektrode 10 im Bereich einer Fixierung 12 dargestellt, bei der das erste Strukturelement 14 aus einem biodegradierbaren Werkstoff geformt ist und als Verbindung zu einem bioresistenten zweiten Strukturelement 28 agiert. Nach dem Abbau des ersten Strukturelementes 14 verbleibt das ankerförmige zweite Strukturelement 28 im Körper, wenn die Elektrode 10 entfernt wird.

Schließlich ist in den Fig. 15a und 15b eine weitere alternative Variante einer Elektrode 10 mit einer Fixierung 12 in einer Draufsicht auf den die Fixierung 12 tragenden Abschnitt der Elektrode 10 bzw. in einem dazugehörigen Querschnitt entlang der Linie A-A dargestellt. Die Fixierung 12 setzt sich, wie in Fig. 15b ersichtlich, aus zwei rohrförmigen ersten und zweiten Strukturelemente 14, 28 zusammen. Das erste Strukturelement 14 ist aus dem biodegradierbaren Werkstoff geformt und so ausgelegt, dass es das zweite Strukturelement 28, das aus einem bioresistenten Material geformt ist, in einem ersten Zustand hält. Das zweite Strukturelement 28 kann demnach einen ersten Zustand einnehmen, in dem es einen geringeren Querschnitt hat und somit eine für die Positionierung günstigere Kontur aufweist. Weiterhin ist das zweite Strukturelement 28 derart ausgelegt, dass es sich selbstexpandierend, d.h. ohne äußeren Zwang, vom ersten Zustand in zumindest einen zweiten Zustand aufweiten kann. Derartige Strukturen können bekannten medizinischen Stützimplantaten aus dem endovaskulären Bereich (Stents) entlehnt werden. Nach dem (weitgehenden) Abbau des ersten Strukturelementes 14 im Körper weitet sich das zweite Strukturelement selbstständig auf. Dieser Vorgang kann dadurch beschleunigt werden, dass über eine elektrische Zuleitung 30, die mit dem ersten Strukturelement 14 über das zweite Strukturelement 28 elektrisch verbunden ist, eine Spannung angelegt wird, die eine galvanische Zersetzung des ersten Strukturelementes 14 bedingt. Als Gegenelektrode kann der Elektrodekopf 24 dienen.

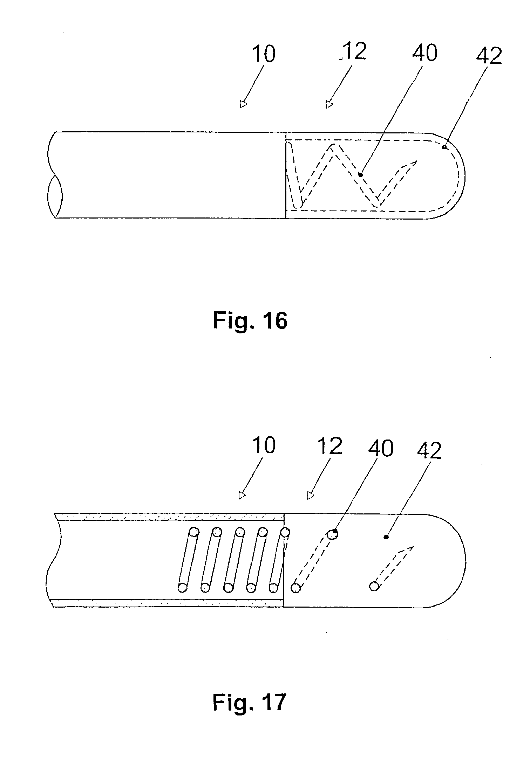

Fig. 16 zeigt die Spitze einer Elektrode 10 mit einer Fixiereinrichtung 12. Die Fixierung 12 umfasst eine Schraubhelix als Strukturelement der Fixierung (entspricht dem aus einem bioresistenten Werkstoff geformten zweiten Strukturelement, der vorangehenden Beispiele; wird hier und in den folgenden Beispielen als Fixierungselement 40 bezeichnet) und eine hohle Kapsel als Schutzelement 42, wobei das Fixierungselement 40 so von der Kapsel umschlossen wird, dass eine atraumatische Einführung durch ein Gefäß oder durch eine kleine Öffnung möglich ist. Das Schutzelement 42 besteht aus einer biodegradierbaren Magnesiumlegierung oben genannter Zusammensetzung, die sich im Körper auflöst und so das Fixierungselement 40 freigibt (das Schutzelement 42 entspricht hier und in den folgenden Beispielen dem ersten Strukturelement, der vorangehenden Beispiele).

Fig. 17 zeigt einen Längsschnitt durch die Spitze einer Elektrode 10 mit einer Fixiereinrichtung 12, bei der eine Schraubhelix als Fixierungselement 40 vorgesehen ist und bei der dieses Fixierungselement 40 vollständig in einen biodegradierbaren Werkstoff aus einer Magnesiumlegierung eingebettet ist, der die Form einer Kapsel einnimmt und als Schutzelement 42 dient.

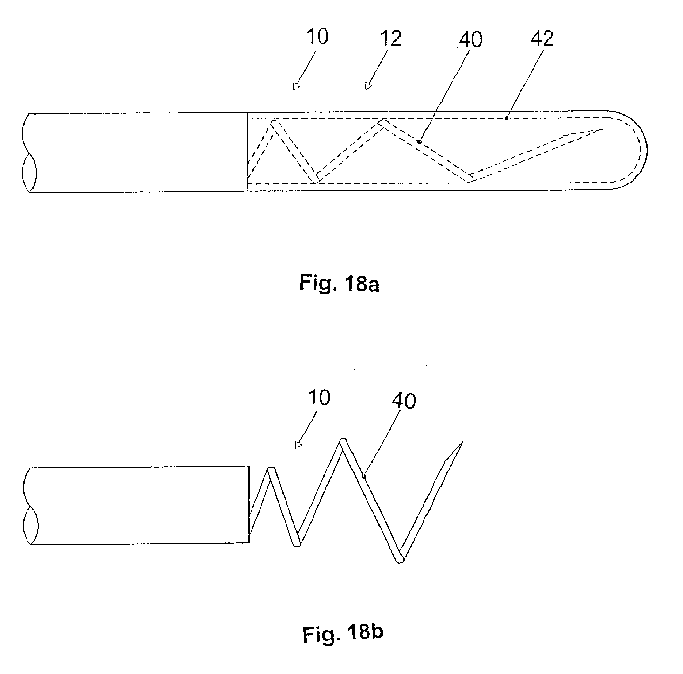

Fig. 18a zeigt die Spitze einer Elektrode 10 mit einer Fixiereinrichtung 12, bei der eine Schraubhelix als Fixierungselement 40 vorgesehen ist, wobei die Schraubhelix durch eine Kapsel als Schutzelement 42 in einer ersten Form gehalten wird, deren Durchmesser kleiner als ein Durchmesser der Schraubhelix in einer entspannten, zweiten Form ist. Mit anderen Worten, das Fixierelement 40 nimmt zunächst eine für den Einführvorgang günstigere erste Form ein. Sobald die Kapsel abgebaut ist geht das Fixierelement 40 in die zweite Form über, die zur Fixierung im umliegenden Gewebe günstiger ist. Fig. 18b zeigt dieselbe Anordnung, nachdem sich das Schutzelement 42 aufgelöst hat.

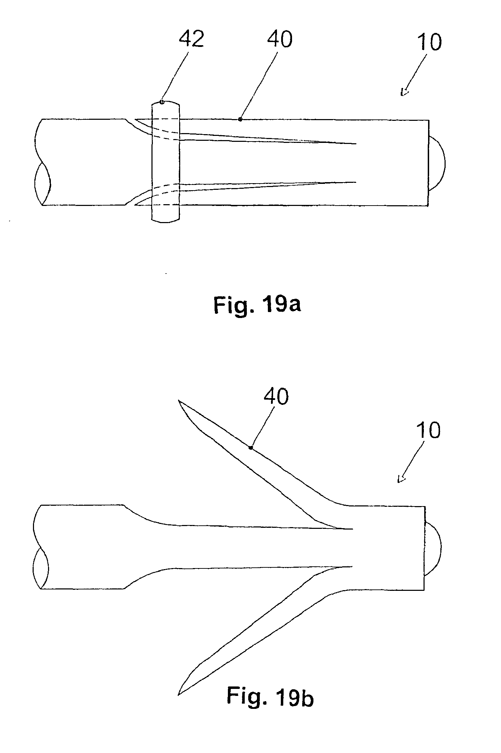

Fig. 19a zeigt die Spitze einer Elektrode 10, bei der Anker als Fixierungselemente 40 vorgesehen sind, die durch einen federnden Bestandteil oder durch inhärente Federeigenschaften ausklappbar sind, d.h. von einer ersten für die Positionierung günstigeren Form in eine zweite für die Fixierung günstigere Form übergehen können, wenn sich das als Band ausgeführte Schutzelement 42 aufgelöst hat. Fig. 19b zeigt dieselbe Anordnung, nachdem sich das Schutzelement 42 aufgelöst hat.

## Patentansprüche

1. Fixierung für implantierbare Elektroden und Katheter, wobei die Fixierung (12, 112) wenigstens ein erstes Strukturelement (14, 114) umfasst, das aus einem biodegradierbaren Werkstoff besteht, **dadurch gekennzeichnet, dass** der Werkstoff eine biodegradierbare Legierung auf Basis von Magnesium ist und die Fixierung (12) eine elektrische Zuleitung (30) umfasst, die mit dem ersten Strukturelement (14, 114) elektrisch leitend verbunden ist und über die an das erste Strukturelement (14, 114) eine elektrische Spannung angelegt werden kann.

2. Fixierung nach Anspruch 1, **dadurch gekennzeichnet, dass** die Legierung eine Magnesiumlegierung ist, die Seltenerdmetalle und Yttrium enthält.

3. Fixierung nach Anspruch 1 oder 2, **dadurch gekennzeichnet, dass** die Legierung eine Magnesiumlegierung mit folgenden Gewichtsanteilen der Legierungskomponenten ist:
- Seltenerdmetalle 2.0 bis 5.0 Gew.% und/oder
- Yttrium 3.5 bis 4.5 Gew.% und/oder
- Neodym 1.5 bis 3.0 Gew.% und/oder
- Zirkonium 0.3 bis 1.0 Gew.% und/oder
- Aluminium < 0.5 Gew.%, insbesondere < 0.01 Gew.%, und/oder
- Rest < 0.5 Gew.%, insbesondere < 0.3 Gew.%,
wobei Magnesium den auf 100 Gew. % verbleibenden Gewichtsanteil an der Legierung einnimmt.

## Claims

1. A fixation means for implantable electrodes and catheters, wherein the fixation means (12, 112) comprises at least one structural element (14, 114) consisting of a biodegradable material, **characterized in that** the material comprises a biodegradable magnesium-based alloy and the fixation means (12) comprises an electrical supply line (30) which is connected to the first structural element (14, 114) in an electrically conductive manner and via which an electrical voltage can be applied to the first structural element (14, 114).

2. The fixation means according to claim 1, **characterized in that** the alloy is a magnesium alloy which contains rare earth metals and yttrium.

3. The fixation means according to claim 1 or claim 2, **characterized in that** the alloy is a magnesium alloy comprising the follow proportions by weight of the alloying components:
- rare earth metal 2.0 to 5.0% by weight and/or
- yttrium 3.5 to 4.5% by weight and/or
- neodymium 1.5 to 3.0% by weight and/or
- zirconium 0.3 to 1.0% by weight and/or
- aluminum <0.5% by weight, in particular <0.01% by weight, and/or
- balance <0.5% by weight, in particular <0.3% by weight,
wherein magnesium accounts for the proportion by weight that remains to 100% by weight of the alloy.

## Revendications

1. Moyen de fixation pour électrodes et cathéters implantables, dans lequel le moyen de fixation (12, 112) comprend au moins un élément structural (14, 114) consistant en une matière biodégradable, **caractérisé par le fait que** le matériau comprend un alliage à base de magnésium biodégradable et que le moyen de fixation (12) comprend une ligne d'alimentation électrique (30) qui est connectée au premier élément structural (14, 114) d'une manière conductrice de l'électricité et par l'intermédiaire de laquelle une tension électrique peut être appliquée au premier élément structural (14, 114).

2. Moyen de fixation selon la revendication 1, **caractérisé par le fait que** l'alliage est un alliage de magnésium qui contient des métaux des terres rares et de l'yttrium.

3. Moyen de fixation selon la revendication 1 ou la revendication 2, **caractérisé par le fait que** l'alliage est un alliage de magnésium comprenant les proportions suivantes en poids des composants d'alliage :
- métal des terres rares 2,0 à 5,0 % en poids et/ou
- yttrium 3,5 à 4,5 % en poids et/ou
- néodyme 1,5 à 3,0 % en poids et/ou
- zirconium 0,3 à 1,0 % en poids et/ou
- aluminium < 0,5 % en poids, en particulier < 0,01% en poids et/ou
- complément < 0,5 % en poids, en particulier < 0,3 % en poids,
le magnésium représentant la proportion en poids qui reste par rapport à 100 % en poids de l'alliage.
